# EUROPEAN PATENT APPLICATION

(11) **EP 2 902 382 A1**
(43) Date of publication of application: **05.08.2015**
(21) Application number: 14382032.2
(22) Date of filing: 30.01.2014
(51) Int. Cl.: C07C 239/10, C07C 239/12, A61K 31/00, A61L 2/00

(54) **Primary hydroxylamines and uses thereof**

(71) Applicant: Fundació Institut de Recerca Biomèdica IRB (Barcelona), 08028 Barcelona (ES); Fundació Institut de Bioenginyeria de Catalunya (IBEC), 08028 Barcelona (ES)
(72) Inventor: Torrents Serra, Eduard, E-08028 Barcelona (ES); Miret Casals, Laia, E-43713 El Papiolet (Sant Jaume dels Domenys) - Tarragona (ES); Abericio Palomera, Fernando, E-08007 Barcelona (ES)
(74) Representative: ABG Patentes, S.L.

(57) **Abstract**

The present invention relates to primary hydroxylamines, to pharmaceutical compositions comprising them, to their use in the treatment and/or prevention of bacterial infections and to their use in the prevention of formation or reduction of formed biofilms of bacterial origin.

## Description

### FIELD OF THE INVENTION

The present invention relates to primary hydroxylamines, to pharmaceutical compositions comprising them, to their use in the treatment and/or prevention of bacterial infections and to their use in the prevention of formation or reduction of formed biofilms of bacterial origin.

### BACKGROUND OF THE INVENTION

The advantages offered by antibiotics in the treatment of infectious diseases are compromised due to the increase in the number of antibiotic-resistant bacterial strains. This reduces the efficiency of antibiotic treatments and poses a serious health and economical problem. Currently, the need for novel antibiotics is becoming increasingly apparent (Davies D., Davies J., Microbiol. Mol. Biol. Rev. 2010, 74(3), 417; Kesselheim A. S., Outterson K., Health Aff. 2010, 29, 1689, Butler MS, Blaskovich MA, Cooper MA., J Antibiot 2013 66(10):571-91, Iwamoto M, Mu Y, Lynfield R, Bulens SN, Nadle J, Aragon D, Petit S, Ray SM, Pediatrics. 2013, 132(4) e817-24, 1: Watkins RR, Papp-Wallace KM, Drawz SM, Bonomo RA., Front Microbiol. 2013;4:392). It has been reported that every year 25000 patients die in the European Union from a bacterial infection which is multiresistent to the presently existing drugs. Thus, there is an urgent need to discover new antibacterial agents against bacteria that have become resistant to other drugs, in particular those directed towards new or under-exploited targets or working through new mechanisms.

Control of bacterial infection using chemotherapeutic principles and antibiotics is based on inhibition of cell wall synthesis, protein synthesis and other metabolic pathways. Since genetic material in the form of DNA is common to all microorganisms, inhibition of DNA synthesis is an attractive principle to suppress bacterial proliferation.

Ribonucleotide reductases are essential enzymes for microorganisms and are responsible for the supply of all four deoxyribonucleotides required for *de novo* DNA synthesis and repair.

Torrents E. et al (Efficient growth inhibition of Bacillus anthracis by knocking out the ribonucleotide reductase tyrosyl radical, PNAS, December 2005, V. 102, Nr. 50, pp 17946-17951) have reported that the tyrosyl radical generated in Ribonucleotide reductases is a possible target for tailored antibacterial drugs and, being extremely sensitive to hydroxylamine and to N-methylhydroxylamine.

Gerez, C. et al. (Reduction of the Small Subunit of E. coli Ribonucleotide Reductase by Hydrazines and Hydroxylamines; Biochemistry, 1992, V. 31, pp 780-786) tested the ability of 6 primary hydroxyl amines (namely, hydroxylamine, *N-*methylhydroxylamine, *N*-isopropylhydroxylamine, *N*-terc-butylhydroxylamine, *N-*phenylhydroxyl-amine and *N*-benzylhydroxylamine) to reduce the redox centers of E. coli ribonucleotide reductase enzyme. The study reported at protein level the rate of reaction in terms of t½ (time required to reduce 50% of the redox centers). The results showed that hydroxylamine, *N*-methylhydroxylamine and *N*-phenylhydroxylamine react faster than *N*-benzylhydroxylamine, *N*-isopropylhydroxylamine and *N*-terc-butylhydroxylamine.

It has also been previously suggested (Torrents E. et al; Antibacterial activity of radical scavengers against class Ib ribonucleotide reductase from Bacillus anthracis, Biol. Chem., 2010, V. 391, pp 229-234) to use hydroxylamine and methyl hydroxylamine as antibacterial agents.

The present invention seeks to provide alternative hydroxylamine compounds showing a spectrum of action spanning both gram-positive and gram-negative bacteria with a high potency to arrest and inhibit bacterial growth and proliferation.

The inventors have now discovered a family of hydroxylamines which are capable of arresting bacterial growth at relatively low concentrations. The compounds of the invention have been shown to have a minimum inhibitory concentration (MIC) against several bacteria (both Gram-positive and Gram-negative), which is substantially lower than the MICs of *N*-benzylhydroxylamine and other hydroxylamines of similar structure. The hydroxylamines of the present invention are particularly effective against *Bacillus anthracis, Staphylococcus epidermis* and *Staphylococcus aureus.*

Additionally the inventors have found that the hydroxylamines of the present invention are capable of inhibiting the formation of bacterial biofilms and to reduce the amount of already formed bacterial biofilms being critical in bacterial chronic infections.

### SUMMARY OF THE INVENTION

In a first aspect, the present invention relates to a compound of formula (I): 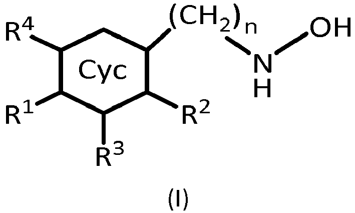 wherein
- the six-membered cycle labeled "Cyc" is selected from the group consisting of benzene and cyclohexane
- n is 1 or 2
- R¹ is selected from the group consisting of hydrogen, fluorine and methoxy group
- R² is selected from the group consisting of hydrogen, methyl group and methoxy group
- R³ is selected from the group consisting of hydrogen and trifluoromethyl group
- R⁴ is selected from the group consisting of hydrogen and trifluoromethyl group
with the proviso that when n is 1 and Cyc is benzene then at least one or R¹, R², R³ and R⁴ is different from hydrogen
or stereoisomers or pharmaceutically acceptable salts thereof,
for use in the treatment or prevention of infections of Ribonucleotide reductase-expressing bacteria.

In a second aspect, the present invention relates to a compound of formula (II): 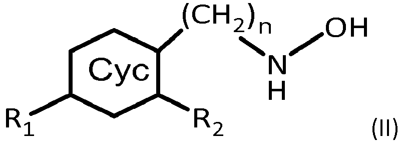 wherein
- the six-membered cycle labeled "Cyc" is selected from the group consisting of benzene and cyclohexane
- n is 1 or 2
- R¹ is selected from the group consisting of fluorine and methoxy group
- R² is selected from the group consisting of methyl group and methoxy group or a stereoisomer or pharmaceutically acceptable salt thereof,
with the proviso that when the six-membered cycle labeled "Cyc" is benzene, then n is 2.

In a third aspect, the present invention relates to a compound of formula (II) as defined in the second aspect or a stereoisomer or pharmaceutically acceptable salt thereof, for use as a medicament.

In a fourth aspect, the present invention relates to a pharmaceutical composition comprising a compound of formula (I) as defined in the first aspect or a stereoisomer or pharmaceutically acceptable salt thereof, and one or more pharmaceutically acceptable excipients.

In a fifth aspect, the present invention relates to the non-therapeutic use of a compound of formula (I) as defined in the first aspect or a stereoisomer or pharmaceutically acceptable salt thereof, to inhibit growth of Ribonucleotide reductase -expressing bacteria outside the human body.

In a sixth aspect, the present invention relates to the non-therapeutic use of a compound of formula (I) as defined in the first aspect or a stereoisomer or pharmaceutically acceptable salt thereof, to inhibit the formation of bacterial biofilms or to reduce the amount of already-formed bacterial biofilms outside the human body.

In a seventh aspect, the present invention relates to the non-therapeutic use of a compound of formula (I) as defined in the first aspect or a stereoisomer or pharmaceutically acceptable salt thereof, to treat a medical device thereby depositing an amount of the said compound onto said device.

### DESCRIPTION OF THE INVENTION

### Description of the Figures

Fig. 1 shows the capacity of various concentrations of *N*-phenethyl-hydroxylamine, *N*-cyclohexylmethyl-hydroxylamine, *N*-(4-fluorobenzyl)-hydroxylamine and *N*-(2,4-dimethoxybenzyl)-hydroxylamine to inhibit *P. aeruginosa* from forming biofilms.
Fig. 2 shows the capacity of various concentrations of *N*-phenethyl-hydroxylamine, *N*-cyclohexylmethyl-hydroxylamine, *N*-(4-fluorobenzyl)-hydroxylamine and *N*-(2,4-dimethoxybenzyl)-hydroxylamine to inhibit *S. aureus* from forming biofilms.
Fig. 3 shows the capacity of various concentrations of *N*-cyclohexylmethyl-hydroxylamine to reduce already formed *P. aeruginosa* biofilms. The effect of two consecutive *N*-cyclohexylmethyl-hydroxylamine treatments (2 days) on formed biofilm is shown.
Fig. 4 shows the capacity of various concentrations of *N*-cyclohexylmethyl-hydroxylamine, *N*-(4-fluorobenzyl)-hydroxylamine and *N*-(2,4-dimethoxybenzyl)-hydroxylamine to reduce already formed *S. aureus* biofilms.
Fig. 5 shows the capacity of various concentrations of N-phenethyl-hydroxylamine to reduce already formed *S. aureus* biofilms. The effect of two consecutive N-phenethyl-hydroxylamine treatments (3 days) on formed biofilm is shown.
   - Fig. 6 shows the capacity of various concentrations of *N*-(3,5-Bis-trifluoromethylbenzyl)-hydroxylamine to reduce already formed *S. aureus* biofilms. The effect of two consecutive *N*-(3,5-Bis-trifluoromethylbenzyl)-hydroxylamine treatments (3 days) on formed biofilm is shown.

### Definitions

As used herein, the term "pharmaceutically acceptable salt" embraces salts with a pharmaceutically acceptable acid or base, which are synthesized from the parent compound which contains an acidic moiety by addition of a pharmaceutically acceptable base, or which are synthesized from the parent compound which contains a basic moiety by addition of a pharmaceutically acceptable acid. Pharmaceutically acceptable acids include both inorganic acids, for example, hydrochloric, sulfuric, phosphoric, diphosphoric, hydrobromic, hydroiodic, and nitric acid, and organic acids, for example, citric, fumaric, maleic, malic, mandelic, ascorbic, oxalic, succinic, tartaric, benzoic, acetic, methanesulfonic (mesylate), ethanesulfonic, benzenesulfonic (besylate), or p-toluenesulfonic (tosylate) acid. Pharmaceutically acceptable bases include alkali metal (e.g., sodium or potassium) and alkali earth metal (e.g., calcium or magnesium) hydroxides and organic bases, such as alkyl amines, arylalkyl amines, and heterocyclic amines. For instance, pharmaceutically acceptable salts of compounds provided herein are synthesized from the parent compound which contains a basic or an acid moiety by conventional chemical methods. Generally, such salts are, for example, prepared by reacting the free base or free acid forms of these compounds with a stoichiometric amount of the appropriate acid or base, respectively, in water or in an organic solvent or in a mixture of the two. Preferably, the pharmaceutically acceptable salt is the mesylate.

As used herein, the term "stereoisomer" makes reference to compounds made up of the same atoms bonded by the same sequence of bonds but having different three-dimensional structures which are not interchangeable, depending on the presence of chiral centers, such as enantiomers, or diastereomers, or depending on the presence of multiple bonds, such as Z and E isomers. The single isomers, enantiomers or diastereoisomers and mixtures thereof fall within the scope of the present invention. If mixtures of stereoisomers are obtained, these isomers may be separated by conventional techniques such as preparative chromatography. If there are chiral centers the compounds may be prepared in racemic form, or individual enantiomers may be prepared either by enantiospecific synthesis or by resolution.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon or ¹⁵N-enriched nitrogen are within the scope of this invention.

The terms "treating" and "treatment", as used herein, mean reversing, alleviating, inhibiting the progress of infections caused by Ribonucleotide reductase-expressing bacteria, or one or more symptoms associated with said diseases.

The terms "preventing" and "prevention", as used herein, means inhibiting the onset of infections caused by Ribonucleotide reductase-expressing bacteria, or one or more symptoms associated with said diseases.

As used herein, the term "medical device" makes reference to an instrument, apparatus, implant or related article that is used to diagnose, prevent, or treat a disease or another medical condition, and which does not achieve its purposes through chemical action within or on the body. In use a medical device will be held into contact with some part of the body.

As used herein, the term "bacterial biofilm" defines a group of bacteria in which bacterial cells stick to each other on a surface. Frequently the bacteria in said biofilms are embedded within a self-produced matrix of extracellular polymeric substance. Bacterial biofilms may form on living or non-living surfaces.

As used herein, the term "chronic pulmonary infection" is used to designate bacterial infections that happen in the lungs, chest, sinuses, nose and throat and that occur repeatedly over time. As used herein the term "chronic pulmonary infection" also encompasses lung conditions which are caused by, aggravated by or associated to bacterial infections of the lung. Examples of chronic pulmonary infections include: tuberculosis, non-tuberculous mycobacterium infection, chronic pneumonia, bronchiectasis, chronic granulomatous disease, cystic fibrosis and chronic obstructive pulmonary disease (COPD).

### Compounds of formula (I) and their pharmacological activity

The compounds of formula (I) of the present invention inhibit the replication of Ribonucleotide reductase -expressing bacteria.

Thus, in the first aspect, the present invention provides a compound of formula (I) as defined above, or a stereoisomer or pharmaceutically acceptable salt thereof, for use in the treatment or prevention of infections caused by Ribonucleotide reductase-expressing bacteria, in particular those selected from the group consisting of *Bacillus anthracis, Staphylococcus aureus, Burkholderia cenocepacea, Staphylococcus epidermidis, Enterococcus faecalis* and *Pseudomonas aeruginosa.*

The present invention also relates to the use of a compound of formula (I) as defined above, or a stereoisomer or pharmaceutically acceptable salt thereof, for manufacturing a medicament for the treatment or prevention of infections caused by Ribonucleotide reductase-expressing bacteria, in particular those selected from the group consisting of *Bacillus anthracis, Staphylococcus aureus, Burkholderia cenocepacea, Staphylococcus epidermidis, Enterococcus faecalis* and *Pseudomonas aeruginosa.*

The invention also relates to a method of treatment or prevention of infections caused by Ribonucleotide reductase-expressing bacteria, in particular those selected from the group consisting of *Bacillus anthracis, Staphylococcus aureus, Burkholderia cenocepacea, Staphylococcus epidermidis, Enterococcus faecalis* and *Pseudomonas aeruginosa,* in a subject in need thereof of, which comprises the administration of a therapeutically effective amount of a compound of formula (I) as defined above, or a stereoisomer or pharmaceutically acceptable salt thereof.

In an embodiment, the present invention provides a compound of formula (I) for use as defined above, wherein the six-membered cycle labeled "Cyc" is selected from the group consisting of benzene and cyclohexane, n is 1 or 2, R¹ is selected from the group consisting of hydrogen, fluorine and methoxy group, R² is selected from the group consisting of hydrogen, methyl group and methoxy group and R³ and R⁴ are both hydrogen atoms.

In an embodiment, the present invention provides a compound of formula (I) for use as defined above, wherein n has a value of 1.

In another embodiment, the present invention provides a compound of formula (I) for use as defined above wherein the six-membered cycled labeled "Cyc" is benzene, n has a value of 1 and R¹ is not hydrogen.

In a particular embodiment, the present invention provides a compound of formula (I) for use as defined above, wherein R¹ and R² are both hydrogen atoms.

In a preferred embodiment, the present invention provides a compound of formula (I) for use as defined above, which is selected from the group consisting of:
- *N*-Phenethyl-hydroxylamine
- *N*-Cyclohexylmethyl-hydroxylamine
- *N*-(4-Fluorobenzyl)-hydroxylamine
- *N*-(2,4-Dimethoxybenzyl)-hydroxylamine
- *N*-(4-Methoxy-2-methylbenzyl)-hydroxylamine
- *N*-(3,5-Bis-trifluoromethylbenzyl)-hydroxylamine
or a stereoisomer or pharmaceutically acceptable salt thereof.

In another aspect the invention relates to the non-therapeutic use of a compound of formula (I) as defined above to inhibit growth of Ribonucleotide reductase -expressing bacteria outside the human body.

In another aspect the invention relates to the non-therapeutic use of a compound of formula (I) as defined above to inhibit the formation of bacterial biofilms or to reduce the amount of already-formed bacterial biofilms outside the human body.

In a particular embodiment of the invention the above mentioned non-therapeutic uses involve Ribonucleotide reductase-expressing bacterium selected from the group consisting of *Bacillus anthracis, Staphylococcus aureus, Burkholderia cenocepacea, Pseudomonas aeruginosa, Staphylococcus epidermidis* and *Enterococcus faecalis.*

In another aspect the invention relates to the non-therapeutic use of a compound of formula (I) as defined above to treat a medical device thereby depositing an amount of the said compound onto said device.

### Compounds of formula (II)

In the second aspect, the present invention provides a compound of formula (II) wherein
- the six-membered cycle labeled "Cyc" is selected from the group consisting of benzene and cyclohexane
- n is 1 or 2
- R¹ is selected from the group consisting of fluorine and methoxy group
- R² is selected from the group consisting of methyl group and methoxy group or a stereoisomer or pharmaceutically acceptable salt thereof,
with the proviso that when the six-membered cycle labeled "Cyc" is benzene, then n is 2.

The compounds of formula (II) fall under the definition of the compounds of formula (I). As explained above, these compounds are inhibitors of the replication of Ribonucleotide reductase -expressing bacteria.

Thus, in another aspect, the present invention provides a compound of formula (II) as defined above, or a stereoisomer or pharmaceutically acceptable salt thereof, for use as a medicament.

The present invention also provides the use of a compound of formula (II) as defined above, or a stereoisomer or pharmaceutically acceptable salt thereof, in the manufacture of a medicament.

The present invention provides a compound of formula (II) as defined above, or a stereoisomer or pharmaceutically acceptable salt thereof, for use in the treatment or prevention of infections caused by Ribonucleotide reductase -expressing bacteria.

The present invention also provides the use of a compound of formula (II) as defined above, or a stereoisomer or pharmaceutically acceptable salt thereof, for manufacturing a medicament for the treatment or prevention of infections caused by Ribonucleotide reductase -expressing bacteria, in particular those selected from the group consisting of *Bacillus anthracis, Staphylococcus aureus, Burkholderia cenocepacea, Staphylococcus epidermidis, Enterococcus faecalis* and *Pseudomonas aeruginosa.*

The invention also relates to a method of treatment or prevention of infections caused by Ribonucleotide reductase -expressing bacteria, in particular those selected from the group consisting of *Bacillus anthracis, Staphylococcus aureus, Burkholderia cenocepacea, Staphylococcus epidermidis, Enterococcus faecalis* and *Pseudomonas aeruginosa,* in a subject in need thereof of, which comprises the administration of a therapeutically effective amount of a compound of formula (II) as defined above, or a stereoisomer or pharmaceutically acceptable salt thereof.

### Pharmaceutical compositions/formulations and administration

In another aspect, the present invention provides a pharmaceutical composition comprising a compound of formula (I) as defined above, or a stereoisomer or pharmaceutically acceptable salt thereof, and one or more pharmaceutically acceptable excipients.

The term "pharmaceutically acceptable excipient" refers to a vehicle, diluent, or adjuvant that is administered with the active ingredient. Such pharmaceutical excipients can be sterile liquids, such as water and oils, including those of petroleum, animal, vegetable, or synthetic origin, such as peanut oil, soybean oil, mineral oil, sesame oil, and similar. Water or saline aqueous solutions and aqueous dextrose and glycerol solutions, particularly for injectable solutions, are preferably used as vehicles. Suitable pharmaceutical vehicles are described in "Remington's Pharmaceutical Sciences" by E.W. Martin, 21st Edition, 2005.

Compounds of the invention may be administered by the oral, sublingual, parenteral, subcutaneous, intramuscular, intravenous, transdermal, intranasal, intraocular, and/or rectal routes. The compounds may be administered alone or in combination with one or more other compounds of the invention or one or more other drugs.

### Synthesis of compounds of formula (I) and formula (II)

The compounds of formula (II) represent a particular subgroup of the compounds of formula (I). Thus, the methods described hereinafter in relation to the preparation of compounds of formula (I) may also be used to prepare compound of formula (II)

Scheme 1 shows the synthetic pathway for obtaining compounds of formula (I):

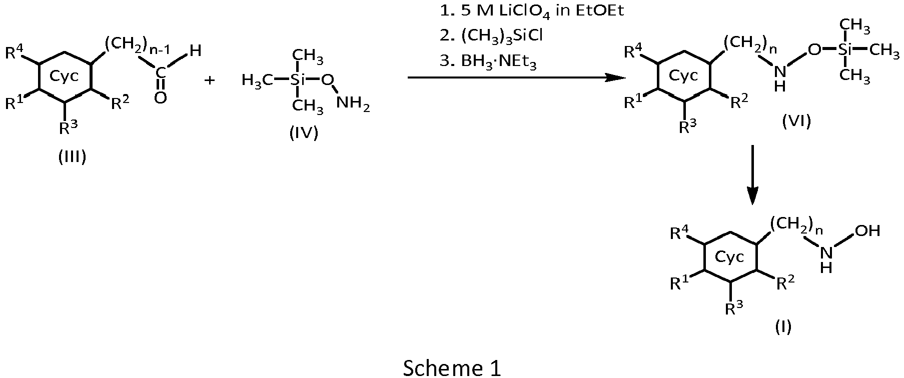

The starting material used for the synthesis compounds of formula (I) according to the present invention is an aldehyde compound of formula (III). These compounds are commercially available or may be synthetized by methods well known to the skilled in the art.

An equivalent of aldehyde of formula (III) is dissolved in a 1.0 to 8.0 M solution of an alkaline perchlorate salt (p.e. LiClO₄) in an organic solvent such as pentane, hexane, cyclohexane, benzene, toluene, chloroform, dichloromethane, tetrahydrofuran and ethyl acetate or diethylether (LPDE solution) and from 1 to 10 equivalents of *O-*trimethylsilylhydroxylamine (IV) are added. The reaction mixture is stirred up to 2 hours and trimethylsilyl chloride is added (1 to 10 equivalents). After stirring the mixture for a period up to 24 hours, a reducing agent selected from diborane, decaborane, borane-tetrahydrofuran (BTHF), borane-dimethyl sulfide (BMS), sodium borohydride, sodium tetrahydroborate and/or borane/triethylamine complex, (i.e. BH₃·NEt_{3;} 1 to 10 equivalents) is added and the reaction mixture is reacted for a period up to 24 hours affording mono *N*-alkyl-*O*-trimethylsilylhydroxylamine. The former steps are carried out in the form of a one pot reaction, i.e. without isolating the products resulting from each reaction step. The reaction is quenched with an aqueous saturated solution of an alkaline salt such as a bicarbonate, sulphate or phosphate (i.e. NaHCO₃) to obtain the mono *N*-alkylhydroxylamine and then extracted with dichloromethane (DCM), another halogen-containing organic solvent or ethyl acetate. The organic extracts are combined, dried (MgSO₄ or other drying agent) and concentrated. Finally, the crude product is flash chromatographed (SiO₂).

The following examples represent specific embodiments of the present invention. They do not intend to limit in any way the scope of the invention defined in the present description.

The following abbreviations are employed herein:
- DCM: dichloromethane
- EtOEt or Et₂O: diethyl ether
- EtOAc: ethyl acetate
- LDPE: lithium perchlorate (LiClO4) in diethyl ether
- NMR: nuclear magnetic resonance
- PBS: Phosphate buffered saline
- TSB: Tryptic soy broth

### Examples

### Materials and methods

### General procedure for the synthesis of hydroxylamines

To a mixture of aldehyde (1 g, 1 eq) in a solution of 5 M LiClO₄/Et₂O (10 mL) was added o-(trimethylsilyl)hydroxylamine (1.5 eq) at room temperature. The mixture was stirred for 10 minutes and (CH₃)₃SiCl (1.5 eq) was added. After stirring the mixture for 8 hours BH₃.NEt₃ (1.5 eq) was added and the mixture was allowed to stir at room temperature overnight.

The reaction was set up for 24 hours and was quenched with aq saturated NaHCO₃ solution, and the product was extracted with DCM. The organics extracts were combined, dried with MgSO₄ and concentrated. The crude product was flash chromatographed (SiO₂) using a hexane-EtOAc gradient from 1:0 to 0:1.

The compounds shown below were synthetized using the method described in the previous section.

¹H, ¹³C and ¹⁹F NMR spectra were recorded on a Varian MERCURY 400 (400 MHz for ¹H NMR, 101 MHz for ¹³C NMR and 376 MHz for ¹⁹F NMR) spectrometer. Chemical shifts (δ) are expressed in parts per million downfield from the deuterated solvent signal was used as reference. Coupling constants are expressed in Hertz.

### Example 1: Synthesis of N-phenethyl-hydroxylamine

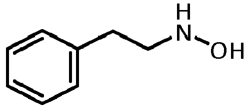

The product was obtained in a 26.3 % yield. ¹H NMR (400 MHz, CDCl₃) δ ppm 7.33-7.26 (m, 2H, ArH), 7.26-7.18 (m, 3H ArH), 4.22-3.92 (m, 2H, NHOH), 3.21 (t, *J* = 7.1 Hz, 2H, CH₂), 2.90 (t, *J* = 7.1 Hz, 2H, CH₂). ¹³C NMR (101 MHz, CDCl₃) δ ppm 139.02 (s), 128.82 (2d), 128.60 (2d), 126.39 (d), 54.75 (t), 33.05 (t).

### Example 2: Synthesis of N-cyclohexylmethyl-hydroxylamine

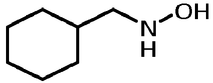

The product was obtained in a 70.2 % yield. ¹H NMR (400 MHz, CDCl₃) δ ppm 6.35 (bs, 2H, NHOH), 2.77 (d, *J* = 6.6 Hz, 2H, CH₂), 1.65 (m, 6H), 1.19 (m, 3H), 0.92 (m, 2H). ¹³C NMR (101 MHz, CDCl₃) δ ppm 60.52 (t), 35.14 (d), 31.32 (2t), 26.50 (t), 25.93 (2t).

### Example 3: Synthesis of N-(4-fluorobenzyl)-hydroxylamine

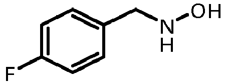

The product was obtained in a 42.4 % yield. ¹H NMR (400 MHz, CDCl₃) δ ppm 7.31 (dd, *J* = 8.4, 5.5 Hz, 2H, ArH), 7.03 (t, *J* = 8.7 Hz, 2H, ArH), 4.01 (s, 2H, CH₂). ¹³C NMR (101 MHz, CDCl₃) 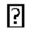 ppm 163.53 (d), 161.081 (s), 130.83 (d), 130.75 (d), 115.38 (d), 115.16 (d), 57.23 (t). ¹⁹F NMR (376 MHz, CDCl₃) δ ppm -114.80 (tt, *J* = 8.8, 5.4 Hz, 1F, CF)

### Example 4: N-(2,4-dimethoxybenzyl)-hydroxylamine

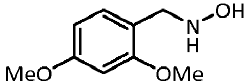

The product was obtained in a 22 % yield. ¹H NMR (400 MHz, CDCl₃) δ ppm 7.16 (d, *J* = 7.9 Hz, 1H, ArH), 6.46-6.43 (m, 2H, ArH), 4.03 (s, 2H, CH₂), 3.82 (s, 3H, CH₃), 3.80 (s, 3H, CH₃). ¹³C NMR (101 MHz, CDCl₃) δ ppm 160.62 (s), 158.78 (s), 131.82 (d), 116.87 (s), 103.83 (d), 98.37 (d), 55.23 (q), 55.12 (q), 53.37 (t).

### Example 5: N-(4-methoxy-2-methylbenzyl)-hydroxylamine

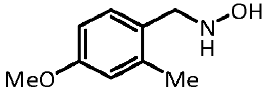

The product was obtained in a 32.8 % yield. ¹H NMR (400 MHz, CDCl₃) δ ppm 7.18 (d, *J* = 8.2 Hz, 1H, ArH), 6.73 (d, *J* = 2.6 Hz, 1H, ArH), 6.70 (dd, *J* = 8.2, 2.767 Hz, 1H, ArH), 4.01 (s, 2H, CH₂), 3.78 (s, 3H, CH₃), 2.35 (s, 3H, CH₃). ¹³C NMR (101 MHz, CDCl₃) δ ppm 158.86 (s), 138.40 (s), 131.28 (d), 126.59 (s), 115.75 (d), 110.78 (d), 54.87 (q), 54.86 (t), 19.07 (q).

### Example 6: N-(3,5-bis-trifluoromethylbenzyl)-hydroxylamine

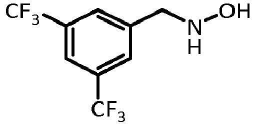

The product was obtained in a26.4 % yield. 1H NMR (400 MHz, CDCL3) δ ppm 7.83 (s, 2H, ArH), 7.82 (s, 1H, ArH), 5.58 (bs, 2H, NHOH), 4.13 (s, 2H, CH2). 13C NMR (101 MHz, CDCL3) δ ppm 140.32 (2s), 131.72 (2q), 129.02 (2d), 124.65 (s), 121.51 (d), 56.873 (t). 19F NMR (376 MHz, CDCL3) δ ppm -62.92 (6F, s, F).
Comparative Example 7: *N*-Benzyl-hydroxylamine hydrochloride

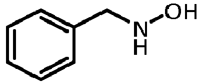

The compound has been obtained from Sigma Aldrich

Comparative Example 8: *N*-(2,6-Dimethoxybenzyl)-hydroxylamine 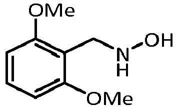 ¹H NMR (400 MHz, DMSO-*d*₆):
The product was obtained in a 32.8 % yield. ¹H NMR (400 MHz, CDCl₃) δ ppm 7.18 (d, *J* = 8.2 Hz, 1H, ArH), 6.73 (d, *J* = 2.6 Hz, 1H, ArH), 6.70 (dd, *J* = 8.2, 2.7 Hz, 1H, ArH), 4.01 (s, 2H, CH₂), 3.78 (s, 3H, CH₃), 2.35 (s, 3H, CH₃). ¹³C NMR (101 MHz, CDCl₃) δ ppm 158.86 (s), 138.40 (s), 131.28 (d), 126.59 (s), 115.75 (d), 110.78 (d), 54.87 (q), 54.86 (t), 19.07 (q).

### Biological Testing

### Bacterial strains and growth conditions

*Wild-type Pseudomonas aeruginosa ATCC 15692 (CECT 4122), Staphylococcus aureus ATCC 12600 (CECT 86)*, *Staphylococcus epidermidis ATCC 1798 (CECT 231), Enterococcus faecalis ATCC 19433 (CECT 481) were obtained from the Spanish Type Culture Collection (CECT) and Bacillus anthracis* Sterne 7700 pXO1-/pXO2- were obtained from the Swedish Defense Research Agency. These strains were stored at -80°C as glycerol stocks. To obtain inocula for examination, the strains were cultured overnight on trypticase soy agar (TSA; Conda-Pronadisa, Spain) or tryptic soy broth (TSB; Sharlau, Spain) medium at 37°C.

### Evaluation of the Minimum inhibitory concentration (MIC)

Minimal inhibitory concentration (MIC) assays were determined by a microtiter broth dilution method as described by Cole et al. (Cole AM, Weis P, Diamond G. J Biol Chem 1997. 272:12008-12013) and modified by Beckloff et al. (Beckloff N, Laube D, Castro T, Furgang D, Park S, Perlin D, Clements D, Tang H, Scott RW, Tew GN, Diamond G. Antimicrob Agents Chemother 2007. 51:4125-4132). In brief, 100 µl of bacteria at a optical density (A₅₅₀) of 0.1 (1x10⁶ cfu/ml) in TSB broth (Sharlau Spain) was inoculated into the wells of 96-well assay plates (tissue culture-treated polystyrene; Nunc™ MicroWell™ Plates with Nunclon™ Delta Surface) in the presence of different concentration of compound in TSB at final concentrations (0, 0.03125, 0.0625, 0.125, 0.25, 0.5, 1, 2, 4 mM). The inoculated microplates were incubated at 37°C for 9 h at 150 rpm in an Infinite 200 Pro microplate reader (Tecan) with 15 min read at OD₅₅₀. Because the water solubility of some compounds is low, we used 50% methanol. Experiments were carried out in triplicate. The MIC was determined as the lowest concentration that completely inhibited bacterial growth.

The MIC values were calculated for each compound. These assays were done in triplicate and the results shown in Table 1 represent the mean value of these triplicates.

Table 1 below shows MIC (minimum inhibitory concentration) value ranges of compounds tested in this assay. Results in table 1 expressed by the following data:
- "A" means 0.625 mM ≤ MIC < 0.125 mM,
- "B" means 0.125 mM ≤ MIC < 0.25 mM,
- "C" means 0.25 mM ≤ MIC < 0.50 mM,
- "D" means 0.50 mM ≤ MIC < 1.00 mM,
- "E" means 1.00 mM ≤ MIC < 2.00 mM,
- "F" means 2.00 mM ≤ MIC < 4.00 mM, and
- "G" means 4.00 mM ≤ MIC
- "n.d.". means not determined

**Table 1**

| **Example** | ***Bacillus anthracis*** | ***Staphylococcus aureus*** | ***Pseudomonas aeruginosa*** | ***Enterococcus faecalis*** | ***Staphylococcus epidermis*** |
|---|---|---|---|---|---|
| 1 | C | C | D | E | n.d. |
| 2 | C | C | B | D | C |
| 3 | A | D | E | D | B |
| 4 | B | D | E | D | B |
| 5 | A | G | E | C | C |
| 6 | A | C | E | D | B |
| Comp 7 | G | G | G | n.d. | n.d. |
| Comp 8 | G | G | G | n.d. | n.d. |

The results summarized in table 1 show that the hydroxylamines of the present invention (Examples 1 to 6) are more potent and/or have a broader spectra of action than similar compounds having a different substitution pattern in the benzene ring (Comparative examples 7 and 8).

### Evaluation of capacity to inhibit the formation of bacterial biofilms

Overnight cultures grown in TSB were diluted to 10⁶ CFU/ml. A 100-µl aliquot was transferred to a 96-well microtiter plate, and 100 µl of different compound concentration, dissolved in TSB, was added. After incubation at 37°C for 48 h without agitation, the bacterial viability was quantified. Essentially after incubation, the medium was removed and the wells were washed three-times with PBS + Polysorbate 20 0.05% and the biofilm attached to the wall of the different wells were scratched off with a sterile tip in 200 µl PBS + Polysorbate 20 0.05%. The suspension was serially diluted and plated onto a TSA plate to measure the viable count (cfu/ml) after incubation.

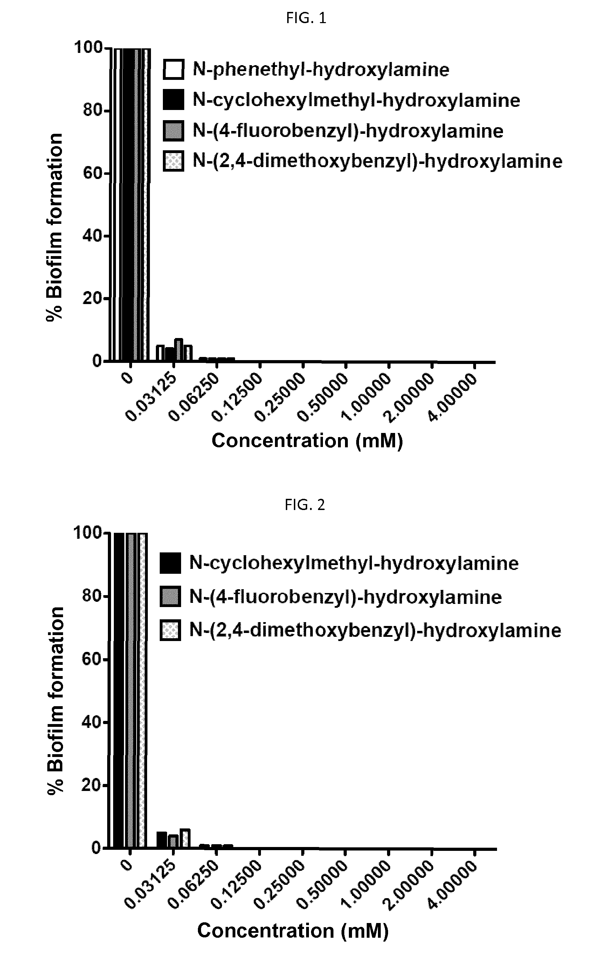

As seen in Fig. 1 and Fig. 2 *N*-phenethyl-hydroxylamine, *N*-cyclohexylmethyl-hydroxylamine, *N*-(4-fluorobenzyl)-hydroxylamine and *N*-(2,4-dimethoxybenzyl)-hydroxylamine inhibit the capacity of *P. aeruginosa* and *S. aureus* biofilm formation.

### Evaluation of capacity to reduce already formed bacterial biofilms

Microtiter plates were filled with 200 µl of 10⁶ CFU/ml in TSB and incubated at 37°C for 24 h or 3 days without shaking. After incubation, the medium was removed and the wells were rinsed twice with 1x phosphate buffered saline (PBS, Gibco) . Different compound concentrations (200 µl) dissolved in TSB were added. After cultivation at 37°C for 24 h, the supernatant was discarded and washed three-times with PBS+Polysorbate 20 0.05% and the biofilm attach to the wall of the different wells were scratched off with a sterile tip in 200 µl PBS + Polysorbate 20 0.05%. The suspension was serially diluted and plated onto a TSA plate to measure the viable count (cfu/ml) after incubation.

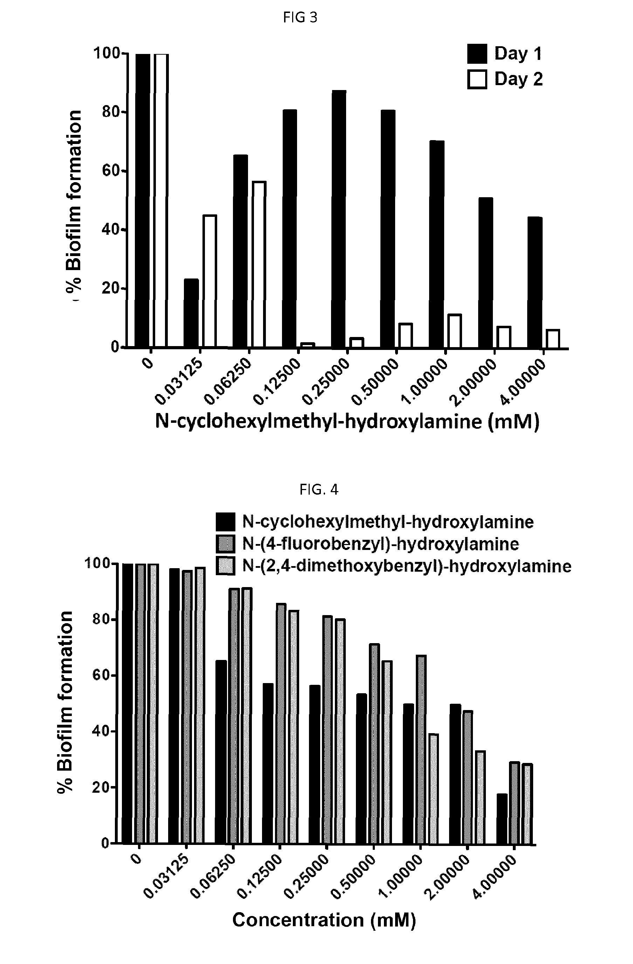

In Fig. 3. the effect of various concentrations of *N*-cyclohexylmethyl-hydroxylamine on *P. aeruginosa* formed biofilm is shown. The effect of two consecutive (2 days) *N*-cyclohexylmethyl-hydroxylamine treatments on formed biofilm is shown.

In Fig. 4. the effect of various concentrations of *N*-cyclohexylmethyl-hydroxylamine, *N*-(4-fluorobenzyl)-hydroxylamine and *N*-(2,4-dimethoxybenzyl)-hydroxylamine on *S. aureus* formed biofilm is shown.

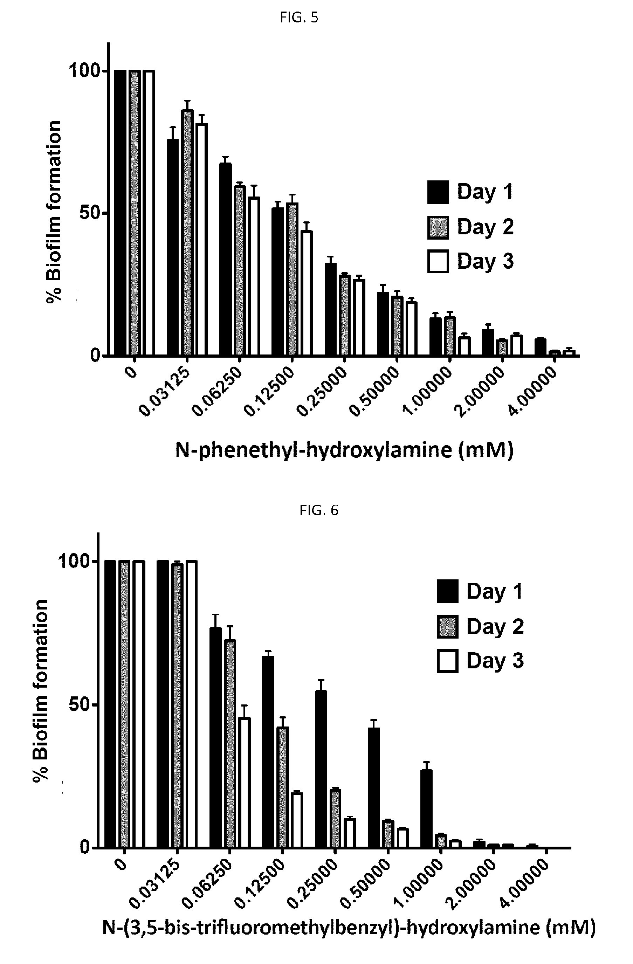

In Fig. 5 the effect of various concentrations of various concentrations of *N-*phenethyl-hydroxylamine on *S. aureus* fomed biofilm is shown. The effect of three consecutive (3 days) *N*-phenethyl-hydroxylamine treatments on formed biofilm is shown.

In Fig. 6 the effect of various concentrations of various concentrations of *N-*(3,5-bis-trifluoromethylbenzyl)-hydroxylamine on *S. aureus* fomed biofilm is shown. The effect of three consecutive (3 days) *N*-(3,5-bis-trifluoromethylbenzyl)-hydroxylamine treatments on formed biofilm is shown.

### Evaluation of cytotoxicity

The murine macrophage J774 cell line was maintained in Dulbecco's Modified Eagle's medium (DMEM) with L-glutamine (Gibco BRL, Grand Island, NY), supplemented with 10% heat-inactivated fetal bovine serum (FBS, Lonza Ltd., Switzerland), containing 100 U/ml penicillin G (Lab ERN, Barcelona, Spain) and 100 µg/ml streptomycin (Lab Reig Jofre, Barcelona, Spain) (complete medium), at 37ºC in a humidified atmosphere with 5% CO₂.

Macrophage (6x10⁴ per well) were seeded onto 96-well tissue culture plates in complete medium without antibiotics, in the presence of different concentration of compound, or left untreated. Cell viability was assessed by using a 3-[4,5-dimethylthiazol-2-yl]-2,5-diphenyltetrazolium bromide (MTT) colorimetric assay (Sigma). After, 24 and 48 h of exposure to the different compounds, culture supernatants were removed and 10% of MTT in complete medium was added to each well and incubated for 3 hours at 37ºC. Then, water-insoluble dark blue formazan was dissolved by adding acidic isopropanol. Absorbance was measured at 550 nm using an ELISA reader (Infinite M200 Microplate Reader, Tecan).

**Table 2**

| **Example** | ***Cytoxicity (IC₅₀) mM*** |
|---|---|
| 1 | 1.89 |
| 2 | 1.33 |
| 3 | 1.30 |
| 4 | 2.27 |
| 5 | 2.18 |
| 6 | 2.07 |
| Comp 7 | >4 |
| Comp 8 | n.d. |

## Claims

1. A compound of formula (I): 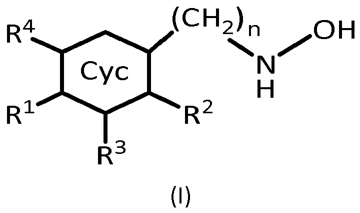 wherein
- the six-membered cycle labeled "Cyc" is selected from the group consisting of benzene and cyclohexane
- n is 1 or 2
- R¹ is selected from the group consisting of hydrogen, fluorine and methoxy group
- R² is selected from the group consisting of hydrogen, methyl group and methoxy group
- R³ is selected from the group consisting of hydrogen and trifluoromethyl group
- R⁴ is selected from the group consisting of hydrogen and trifluoromethyl group
with the proviso that when n is 1 and Cyc is benzene then at least one or R¹, R², R³ and
R⁴ is different from hydrogen
or stereoisomers or pharmaceutically acceptable salts thereof,
for use in the treatment or prevention of infections of Ribonucleotide reductase-expressing bacteria.

2. The compound of formula (I) for use according to claim 1, wherein R³ and R⁴ are both hydrogen atoms.

3. The compound of formula (I) for use according to any one of the preceding claims, wherein n has a value of 1.

4. The compound of formula (I) for use according to any one of the preceding claims, wherein R¹ and R² are both hydrogen atoms.

5. The compound of formula (I) for use according to claim 1 which is selected from the group consisting of:
- *N*-Phenethyl-hydroxylamine
- *N*-Cyclohexylmethyl-hydroxylamine
- *N*-(4-Fluorobenzyl)-hydroxylamine
- *N*-(2,4-Dimethoxybenzyl)-hydroxylamine
- *N*-(4-Methoxy-2-methylbenzyl)-hydroxylamine
- *N*-(3,5-Bis-trifluoromethylbenzyl)-hydroxylamine
or a stereoisomer or pharmaceutically acceptable salt thereof.

6. The compound of formula (I) for use according to any one of the preceding claims wherein the treatment or prevention of infections is carried out in patient suffering from a chronic pulmonary infection.

7. The compound of formula (I) for use according to any one of the preceding claims, wherein Ribonucleotide reductase-expressing bacteria are selected from the group consisting of *Bacillus anthracis, Staphylococcus aureus, Burkholderia cenocepacea, Staphylococcus epidermidis, Enterococcus faecalis* and *Pseudomonas aeruginosa.*

8. A compound of formula (II): 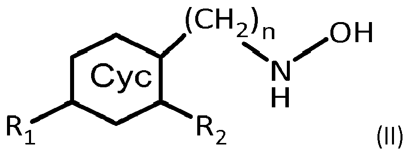 wherein
- the six-membered cycle labeled "Cyc" is selected from the group consisting of benzene and cyclohexane
- n is 1 or 2
- R¹ is selected from the group consisting of fluorine and methoxy group
- R² is selected from the group consisting of methyl group and methoxy group or a stereoisomer or pharmaceutically acceptable salt thereof,
with the proviso that when the six-membered cycle labeled "Cyc" is benzene, then n is 2.

9. A compound of formula (II) as defined in claim 8 or a stereoisomer or pharmaceutically acceptable salt thereof, for use as a medicament.

10. A pharmaceutical composition comprising a compound of formula (I) as defined in any one of claims 1 to 5 or a stereoisomer or pharmaceutically acceptable salt thereof, and one or more pharmaceutically acceptable excipients.

11. The non-therapeutic use of a compound of formula (I) as defined in any one of claims 1 to 5 or a stereoisomer or pharmaceutically acceptable salt thereof, to inhibit growth of Ribonucleotide reductase -expressing bacteria outside the human body.

12. The non-therapeutic use of a compound of formula (I) as defined in any one of claims 1 to 5 or a stereoisomer or pharmaceutically acceptable salt thereof, to inhibit the formation of bacterial biofilms or to reduce the amount of already-formed bacterial biofilms outside the human body.

13. The non-therapeutic use of a compound of formula (I) as defined in any one of claims 1 to 5 or a stereoisomer or pharmaceutically acceptable salt thereof, to treat a medical device thereby depositing an amount of the said compound onto said device.

14. The non-therapeutic use according to any one of claims 11 to 13, wherein the Ribonucleotide reductase-expressing bacterium is selected from the group consisting of *Bacillus anthracis, Staphylococcus aureus, Burkholderia cenocepacea, Pseudomonas aeruginosa, Staphylococcus epidermidis* and *Enterococcus faecalis.*
